# EUROPEAN PATENT SPECIFICATION

(11) **EP 1 383 545 B1**
(45) Date of publication and mention of the grant of the patent: **02.11.2005**
(21) Application number: 02769316.7
(22) Date of filing: 03.05.2002
(51) Int. Cl.: A61L 2/07, A61L 2/26, A61M 5/00, B65B 55/02, C08F 6/00

(54) **METHOD OF REMOVING REFRACTIVE DEFECTS IN CYCLIC OLEFIN MEDICAL DEVICES**
VERFAHREN ZUR ENTFERNUNG REFRAKTIVER DEFEKTE IN MEDIZINISCHEN VORRICHTUNGEN MIT ZYKLISCHEN OLEFINEN
PROCEDE D'ELIMINATION DE VICES DE REFRACTION DANS DES INSTRUMENTS MEDICAUX COMPRENANT UN ELEMENT OLEFINIQUE CYCLIQUE

(30) Priority: 04.05.2001 US 849090
(43) Date of publication of application: 28.01.2004
(73) Proprietor: Becton Dickinson and Company, Franklin Lakes, New Jersey 07417 (US)
(72) Inventor: HETZLER, Kevin, G., Sparta, NJ 07871 (US)
(74) Representative: Weber, Thomas
(86) International application number: PCT/US2002/013941
(87) International publication number: WO 2002/089860

(56) References cited:
- EP-A- 0 418 079
- EP-A- 0 559 146
- US-B1- 6 193 688

## Description

### FIELD OF THE INVENTION

This invention relates to a method of removing refractive defects formed in the matrix of a clear or transparent cyclic olefin component of a medical device during steam sterilization, particularly including terminal sterilization of a prefilled syringe or cartridge in an autoclave for example.

### BACKGROUND OF THE INVENTION

The development of cyclic olefin polymers has suggested the use of such polymers for manufacture of medical devices because medical devices formed of such polymers have good transparency and favorable properties including chemical resistance. Thus, a medical transfer or storage device formed of a cyclic olefin polymer, copolymer or blend will not absorb or dissolve medicaments, vaccines or drugs and such medical devices may be sterilized in an autoclave, for example, without structural damage or chemical interaction with the medicament, drug or vaccine contained therein.

However, it has been found that polycyclicolefin components exhibit visual aberrations following autoclaving. These aberrations appear as a haze, microvoids, sparkles, inclusions or irregularities in the matrix of the polycyclicolefin part and are especially noticeable when illuminated by a bright light in front of a dark background. These refractive defects in the matrix of the polycyclicolefin components hinder drug companies, for example, from inspecting the medicament, drug or vaccine contained in a storage or delivery device, such as a vial, syringe, cartridge or the like. Such storage or delivery devices are inspected by drug companies for particulates in the medicament, drug or vaccine and the refractive defects in the matrix of the medical storage or delivery device interferes with such inspection. Also, health care workers generally visually inspect such medical containers and delivery devices prior to use and may reject such a device having refractive defects because the content appears cloudy upon visual inspection.

The phenomena which creates these refractive defects in the matrix of a polycyclicolefin device or component is not fully understood. However, it is now believed by the Applicant that these refractive defects may result from absorption or adsorption of moisture by the polycyclicolefin during sterilization in the presence of steam, for example, in an autoclave. Polycyclicolefins have an excellent moisture barrier, low extractables, and are as clear or transparent as glass prior to autoclaving. However, following autoclaving, refractive defects appear in the matrix of the polycyclicolefin in the form of a haze, sparkles, inclusions or the like. As stated above, such refractive defects restrict the use of polycyclicolefins for medical containers or delivery devices which must be inspected following steam sterilization.

Terminal sterilization is used by drug manufacturers and others primarily to sterilize a medical delivery device, such as a syringe, cartridge or other delivery device after filling the delivery device with a saline solution, medicament, drug or vaccine. The medical delivery device is first filled with liquid and then steam-sterilized at a temperature of about 120° to 125°C. Such drug delivery devices may be terminally sterilized in an autoclave, which is a sealed container or cabinet; and steam, hot water, or superheated steam is introduced into the autoclave by various methods known by those skilled in this art. A syringe assembly generally includes a barrel, which may be formed of glass or plastic having a reduced diameter tip portion generally including a needle cannula, but which may include a threaded connection referred to as a Luer connector and an open end. A stopper, generally formed of an elastomeric material, is received in the open end, and a plunger is attached to the stopper for delivery of a medicament, drug or vaccine through the cannula. A medical cartridge has a similar construction, but does not include a plunger. During terminal sterilization, the prefilled syringe or cartridge is placed in an autoclave and steam sterilized for 30 to 50 minutes.

However, as set forth above, where the barrel of the syringe or cartridge is formed of a cyclic olefin polymer, copolymer or blend, refractive defects are formed in the matrix of the polycyclicolefin during steam sterilization. There is, therefore, a need to remove the refractive defects formed in the matrix of the cyclic olefin barrel following steam sterilization, particularly where the barrel or component is subject to visual inspection.

### SUMMARY OF THE INVENTION

The method of removing refractive defects formed in the matrix of a clear cyclic olefin component of a medical device during sterilization of this invention begins with steam sterilization, wherein the cyclic olefin component is heated to a first temperature greater than 100°C in the presence of steam for at least 30 minutes to sterilize the cyclic olefin component. As set forth above, it has been found by the Applicant that the refractive defects are formed in the matrix of the cyclic olefin component during this steam sterilization step. The next step is to reduce the temperature of the cyclic olefin component to a second temperature of at least 80°C and maintain the second temperature for at least 20 minutes, preferably in a relatively "dry atmosphere" for preferably at least 40 minutes. Alternatively, the temperature of the cyclic olefin component may be slowly reduced from the steam sterilization temperature to a second temperature, such as ambient, over an extended period of time such as three hours. This second step removes or dissipates the refractive defects formed in the matrix of the cyclic olefin component, returning the component to its original clear or transparent state.

One preferred method of this invention is to first heat the cyclic olefin component in the presence of steam in an autoclave at a temperature of between 120°C and 130°C for 30 to 50 minutes. This results in sterilization of the polycyclicolefin medical component as described above. The medical component is then removed from the autoclave and transferred to a conventional oven having a relatively dry atmosphere and maintaining the second temperature for preferably at least 40 minutes. Alternatively, the entire procedure may be performed in an autoclave, wherein the medical component is first steam or terminally sterilized as described above, then the introduction of water or steam to the autoclave is stopped and the autoclave is preferably vented to reduce the humidity in the autoclave to generally ambient humidity, preferably less than 70% or more preferably less than 50% relative humidity, and the medical component is maintained at the second temperature for at least 20 minutes, more preferably at least 40 minutes. Although not preferred from a commercial processing standpoint, it has been found by the applicant that it is also possible to remove the refractive defects by turning off the autoclave and allowing the component to slowly cool to ambient temperature without venting the autoclave.

As stated above, the reasons why refractive defects form in the matrix of a component formed of a polycyclicolefin during steam sterilization is not fully understood. It is believed, however, that moisture may be absorbed or adsorbed into the polycyclicolefin during steam sterilization. In view of the fact that the refractive defects appear to be in the matrix of the polycyclicolefin, it is believed that moisture may be absorbed by the polycyclicolefin during steam sterilization. Thus, in the preferred method of removing refractive defects formed in the matrix of a clear cyclic olefin component of this invention, the temperature of the cyclic olefin component is maintained at a temperature of at least 70°C following steam sterilization.

The preferred temperature for the second step of the method of this invention, wherein the polycyclicolefin component is maintained at the second temperature, preferably at a reduced humidity, will depend upon the component. Where the polycyclicolefin component is a container or a delivery device, such as a medical vial, syringe or cartridge filled with a liquid, such as a medicament, drug or vaccine, the second temperature should be less than 100°C to prevent boiling of the liquid. However, where the polycyclicolefin component is dry, the second temperature may be between 80°C and 125°C.

As used herein, the terms "cyclic olefin" and "polycyclicolefin" are intended to broadly cover cyclic olefin polymers, copolymers and polymer blends and which may also include various additives. However, as discussed below, it has also been found more difficult to remove refractive defects from certain cyclic olefin polymers than others. As discussed further below, the method of this invention has been found to be particularly advantageous for removing refractive defects from cyclic olefin polymer blends as disclosed, for example, in U.S. Patent No. 5,468,803 of Nippon Zeon Co. Ltd.

### BRIEF DESCRIPTION OF THE DRAWINGS

Figure 1 is a flow chart of one embodiment of the method of this invention;

Figure 2 schematically illustrates filling a conventional syringe barrel;

Figure 3 schematically illustrates insertion of the stopper and plunger assembly;

Figure 4 schematically illustrates steam sterilization in an autoclave, for example;

Figure 5 schematically illustrates the final step in the method illustrated in Figure 1;

Figure 6 is a flow chart illustrating an alternative embodiment of the method of this invention;

Figure 7 schematically illustrates a step in the method of Figure 6; and

Figure 8 schematically illustrates the final step in the method of Figure 6.

### DETAILED DESCRIPTION OF A PREFERRED EMBODIMENT

As set forth above, the method of this invention relates to the removal or elimination of refractive defects in the matrix of a component of a medical device formed of a clear cyclic olefin polymer, copolymer or polymer blend. The method of this invention is particularly adapted for medical storage or delivery devices including vials, syringes, cartridges and the like, wherein the vial or barrel is formed of a cyclic olefin polymer, copolymer or polymer blend which requires steam sterilization, particularly including terminal sterilization where the content of the medical storage or delivery device is visually inspected following terminal sterilization. As will be understood from the following description, however, the method of this invention may also be utilized to remove refractive defects in the matrix of any component of a medical device formed of a clear polycyclicolefin following steam sterilization.

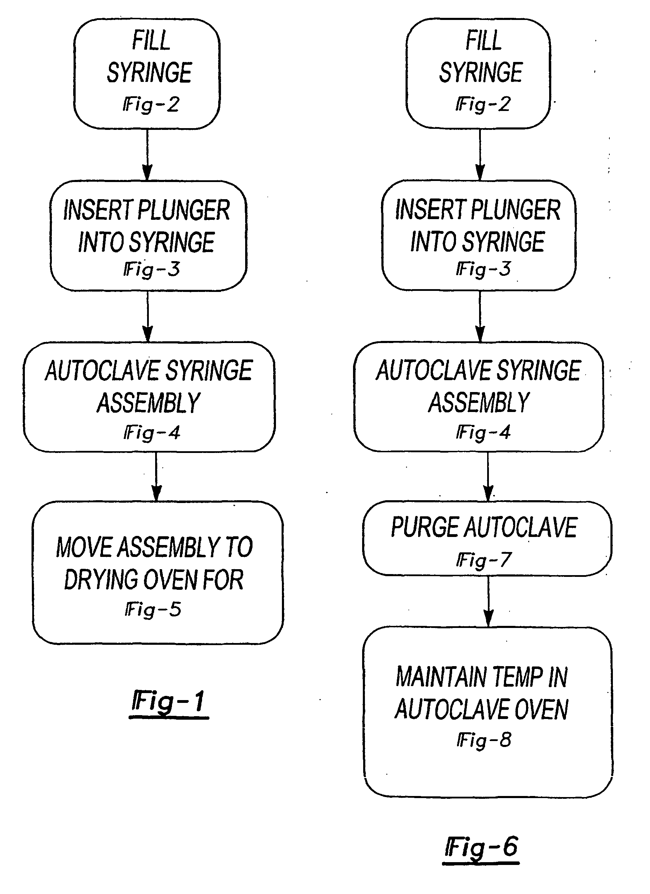

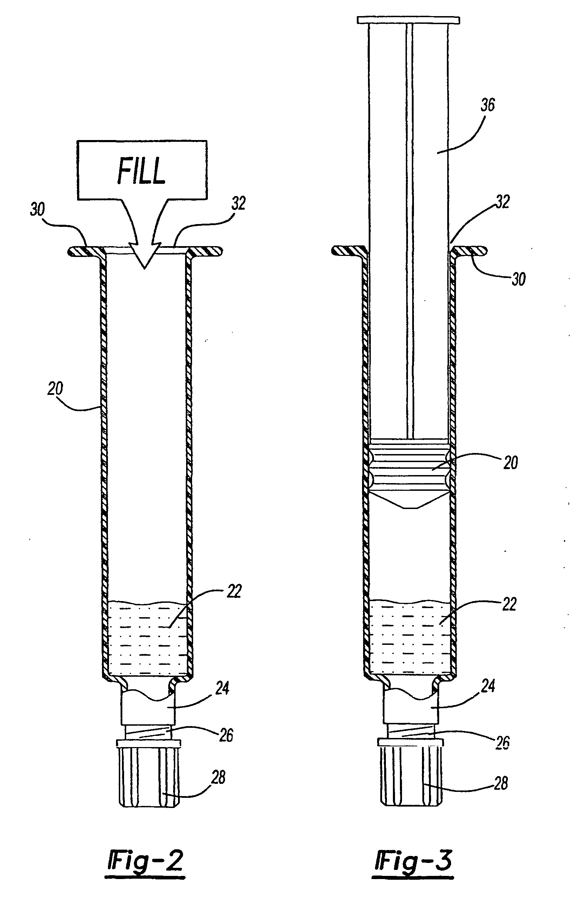

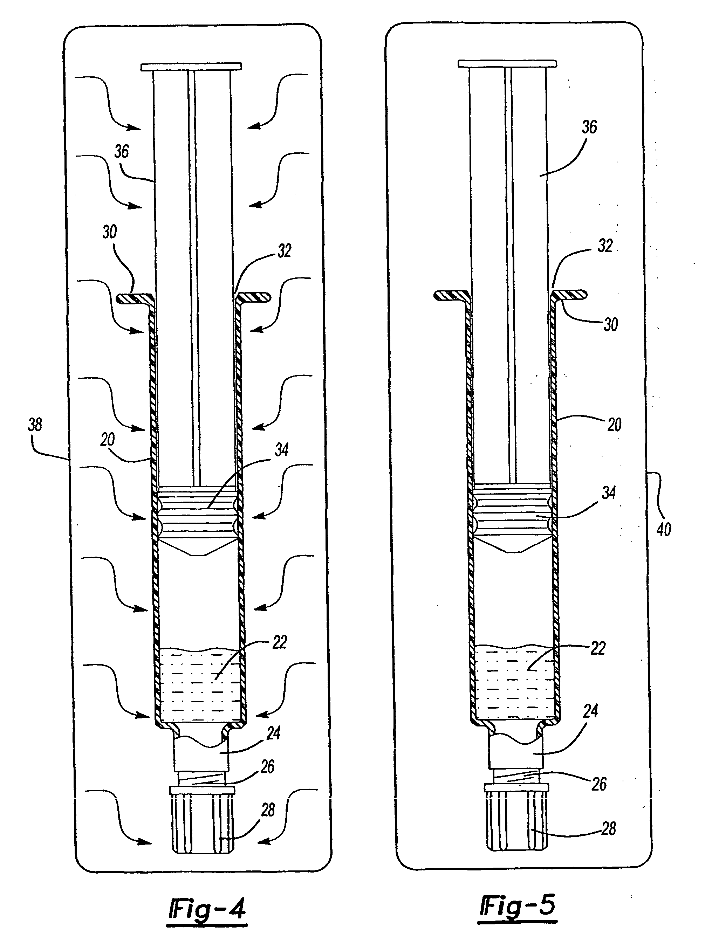

Figures 1 to 5 illustrate one embodiment of the method of this invention with terminal sterilization of a prefilled syringe assembly. As set forth above, however, the same method can be used for other medical storage or transfer devices including cartridges, vials, etc. In the method disclosed, the syringe barrel 20 is filled with a predetermined volume of liquid 22, such as a saline solution, medicament, drug or vaccine as shown in Figure 2. The disclosed embodiment of the syringe barrel includes an integral reduced diameter tip portion 24 having a threaded end 26 which receives a cap 28 sealing the end of the tip portion. Alternatively, the tip portion may include a needle cannula adhesively bonded to or threaded onto the end of the reduced diameter tip portion 24, wherein the tip portion is sealed with a needle shield or sheath (not shown). A conventional syringe barrel also includes a flange 30 at the open end 32. Following the filling of the syringe barrel 20 as shown in Figure 2, the open end 32 is sealed with a stopper and plunger assembly through the open end 32 as shown in Figure 3. The plunger and stopper assembly shown in Figure 3 includes a stopper 34 generally formed of an elastomeric material and a plunger 36 fixed to the stopper 34 by a suitable fastening means including adhesive, a threaded connection, etc. forming a sealed syringe assembly as shown in Figure 3.

The next step in the method of this invention includes steam sterilization in an enclosure 38 as shown in Figure 4, such as the cabinet of an autoclave. As will be understood by those skilled in this art, there are various types of autoclaves wherein steam, superheated steam or hot water and air are introduced into the autoclave cabinet, heating the content of the autoclave to a temperature of greater than 100°C, generally between 120°C and 125°C. Thus, the syringe assembly is heated in the autoclave in the presence of steam to a temperature greater than 100°C or preferably greater than 120°C as shown in Figure 4. The syringe assembly is maintained at the sterilization temperature for at least 20 minutes, more preferably between 30 and 50 minutes, sterilizing the syringe assembly including the liquid 22 in the syringe barrel 20.

The barrel 20 of the syringe illustrated in the figures is formed of a clear or transparent cyclic olefin. As used herein, the term "cyclic olefin" is intended to broadly cover cyclic olefin polymers, copolymers, and blends. Polycyclicolefins are also known as polycycloolefins. As set forth above, refractive defects are formed in the matrix of the cyclic olefin barrel 20 during steam sterilization in the form of a haze, microvoids, sparkles, inclusions or irregularities. These refractive defects interfere with visual inspection of the liquid 22 in the barrel 20, particularly visual inspection of the liquid for particulates. Health care workers may also reject a prefilled syringe where the liquid 22 appears cloudy because of the refractive defects in the matrix of the polycyclicolefin barrel 20. It is therefore very desirable to remove these refractive defects from the clear cyclic olefin barrel 20.

The method of removing the refractive defects in the polycyclicolefin barrel 20 then includes heating the prefilled syringe at a second temperature preferably less than the boiling temperature of the liquid 22 in the barrel or less than 100°C preferably at a reduced humidity compared to the humidity of the autoclave. One method of performing this step is to remove the syringe assembly from the autoclave 38 and place the syringe assembly in a conventional oven or other heated enclosure having a relatively low humidity as shown at 40 in Figure 5. The syringe assembly is then maintained at the second temperature for at least 20 minutes or the temperature is slowly reduced such that the temperature of the syringe assembly is maintained above 70°C for at least 40 minutes. It has been found that the temperature in the second enclosure 40 should be maintained at least 80°C preferably for at least 40 minutes to substantially fully remove the refractive defects formed in the matrix of the clear cyclic olefin barrel 20. Thus, the preferred range of temperatures in the second enclosure 40 is between about 80°C and 100°C for a prefilled syringe following terminal sterilization.

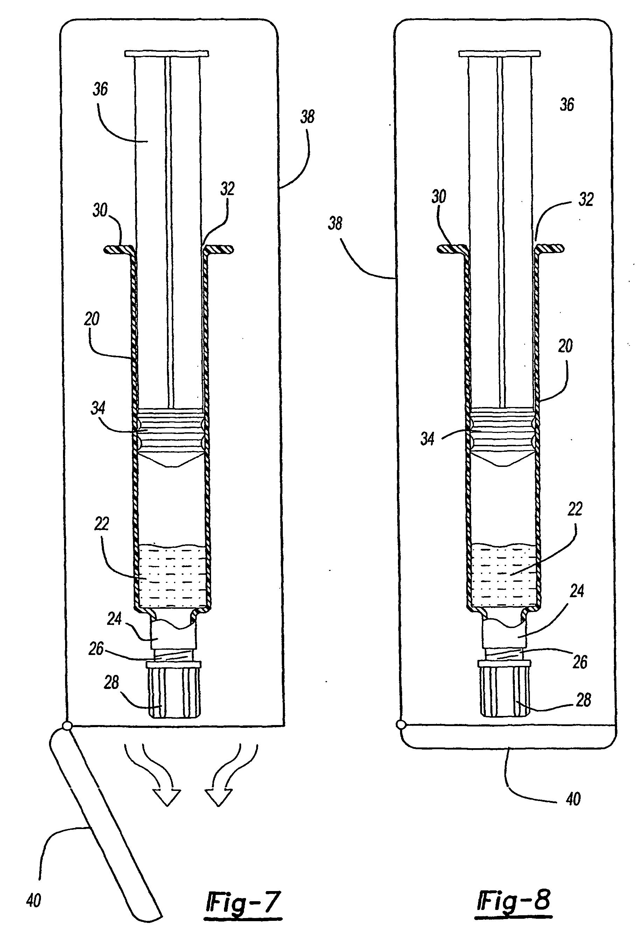

Figure 6 illustrates an alternative method of this invention, wherein the steam sterilization and removal of the refractive defects is performed in the autoclave. This method first includes filling the syringe barrel 20 with a liquid 22 as shown in Figure 2 and described above. The syringe barrel is then sealed with a stopper 34 as shown in Figure 4 as described above. Next, prefilled syringe assembly is steam sterilized in an autoclave 38, for example, as described above in regard to Figure 4. Next, the autoclave is vented by opening vent 40 to remove the steam as schematically illustrated in Figure 7 and the water or steam lines to the autoclave are shut off. Finally, the prefilled syringe assembly is heated at the second temperature, preferably between 80°C and 100°C as described above in the autoclave 38 as shown in Figure 8. The syringe assembly is thus heated in a relatively dry atmosphere, preferably having a relative humidity of less than 50% for at least 20 minutes, or more preferably at least 40 minutes. Alternatively, the autoclave is turned off and the prefilled syringe is permitted to slowly cool to ambient without venting such that the syringe is maintained at a temperature greater than 70°C for at least 40 minutes.

As set forth above, the method of this invention may also be utilized to remove refractive defects formed in the matrix of a clear olefin component of a medical storage or transfer device, wherein the medical storage or delivery device is not prefilled. For example, the method of this invention might be used to remove refractive defects in a cyclic olefin syringe barrel, cartridge or vial following steam sterilization. Where the medical storage or delivery device does not include a liquid, the second temperature may be greater than 100°C, wherein the dry cyclic olefin component is heated in a relatively dry atmosphere as described above. In this method, the preferred range for the second temperature is between 80°C and 127°C. As will be understood, however, there are other methods of sterilizing dry medical components which do not require steam. Therefore, the method of this invention is most preferred for terminal sterilization of prefilled medical components which requires terminal steam sterilization.

As set forth above, the method of this invention is particularly adapted for removing refractive defects formed in the matrix of a clear cyclic olefin component of a medical device. It has been found, however, that the method of this invention is particularly suitable for removing refractive defects formed in the matrix of a clear cyclic olefin thermoplastic such as Zeonex™ available from Zeon Chemicals of Louisville, Kentucky. The cyclic olefin thermoplastic utilized in the test conducted by the Applicant was Zeonex 690R. It is understood that Zeonex™ is a polymer composition including a thermoplastic norbornene polymer or norbornene polymer blend with a rubber-like polymer as set forth in U.S. Patent No. 5,468,803 and U.S. Patent No. 5,561,208 the disclosures of which are incorporated herein. Although cyclic olefin has been broadly defined above, it has been found by the Applicant that the method of this invention was not successful in removing refractive defects in all cyclic olefins. Specifically, the method of this invention did not remove refractive defects formed in the matrix of syringe barrels formed of Topas™, which is a cyclic olefin copolymer with polyethylene available from the Ticona Division of Celanese AG. The reason why this method was not successful with Topas™ cyclic olefin is not presently understood.

As will be understood by those skilled in this art, various modifications may be made to the method of this invention within the purview of the appended claims. Although the method of this invention was particularly developed for removing refractive defects in a prefilled medical container or delivery device formed of a cyclic olefin copolymer blend following terminal sterilization, the method of this invention may also be utilized for other polycyclicolefin components following steam sterilization. In the preferred embodiment of the method of this invention, the temperature of the cyclic olefin component is maintained at or slowly reduced to a temperature of greater than 70°C prior to heating at the second temperature preferably in a relatively dry atmosphere. As will thus be understood, the step of maintaining the cyclic olefin component at the second temperature may be accomplished either by quickly reducing the temperature from the steam sterilization temperature to the second temperature, such as by moving the component to an oven, or by slowly reducing the temperature in the autoclave for example, whereby the component is maintained at a temperature greater than 70°C or more preferably greater than 80°C for at least 20 minutes or more preferably greater than 30 minutes. The removal of the refractive defects from the clear cyclic olefin component following steam sterilization opens the use of such polymers for medical containers and delivery devices, particularly the barrels of syringes and cartridges. Having described the preferred embodiments of the method of this invention, it is now claimed as follows.

## Claims

1. A method of removing refractive defects formed in the matrix of a clear cyclic olefin component of a medical device during sterilization, said method comprising the following steps performed in sequence:
first heating said cyclic olefin component to a first temperature greater than 100°C in the presence of steam for at least 30 minutes to sterilize said cyclic olefin component; and
in the next step reducing the temperature of said cyclic olefin component to a second temperature of at least 80 °C and maintain said second temperature for at least 20 minutes to remove said refractive defects from the matrix of said cyclic olefin component;
under the proviso that a cyclic olefin copolymer with polyethylene is excluded from the cyclic olefin component.

2. The method of removing refractive defects formed in the matrix of a clear cyclic olefin component as defined in Claim 1, wherein said method includes maintaining said cyclic olefin component at said second temperature in a dry atmosphere relative to said first heating step.

3. The method of removing refractive defects in the matrix of a cyclic olefin component as defined in Claim 1, wherein said method includes heating said cyclic olefin component to said first temperature of greater than 100°C in the presence of steam in an autoclave, then removing said cyclic olefin component from said autoclave and transferring said cyclic olefin component to an oven having a relatively dry atmosphere and maintaining said second temperature in said oven for at least 40 minutes.

4. The method of removing refractive defects in the matrix of a cyclic olefin component as defined in Claim 1, wherein said method includes heating said cyclic olefin component to said first temperature in an autoclave, then reducing the temperature in said autoclave to said second temperature and maintaining said temperature in said autoclave for at least 30 minutes.

5. The method of removing refractive defects in the matrix of a cyclic olefin component as defined in Claim 1, wherein said component is a dry medical container and said method includes heating said dry medical container in the presence of steam in an autoclave, wherein said first temperature is between 120°C to 130°C to sterilize said dry medical container, then reducing said temperature of said dry medical component to said second temperature, wherein said second temperature is between 80°C and 120°C.

6. The method of removing refractive defects in the matrix of a cyclic olefin component as defined in Claim 1, wherein said component is a medical container containing a liquid and said method includes heating said medical container and liquid in the presence of steam in an autoclave for at least 30 minutes, wherein said first temperature is between 120°C to 130°C, and then reducing the temperature of said container and liquid to said second temperature, wherein said second temperature is between 80°C and 100°C.

7. The method of removing refractive defects in the matrix of a cyclic olefin component as defined in Claim 6, wherein said method includes maintaining said medical container and liquid at said second temperature in a relatively dry atmosphere by discontinuing introduction of water into said autoclave.

8. The method of removing refractive defects in the matrix of a cyclic olefin component as defined in Claim 1, wherein said component is a syringe barrel formed of a cyclic olefin polymer or copolymer, said method including sterilizing said cyclic olefin barrel in an autoclave at said first temperature, wherein said first temperature is between 120°C and 130°C, then reducing the temperature to said second temperature, wherein said second temperature is between 80°C and 120°C and maintaining said second temperature.

## Patentansprüche

1. Verfahren zur Entfernung von Brechungsfehlern, die während der Sterilisation in der Matrix einer klaren Cycloolefin-Komponente einer medizinischen Vorrichtung entstehen, wobei das Verfahren die folgenden Schritte umfasst, die nacheinander durchgeführt werden:
zuerst Erhitzen der Cycloolefin-Komponente auf eine erste Temperatur von über 100 °C in Gegenwart von Dampf während wenigstens 30 Minuten, so dass die Cycloolefin-Komponente sterilisiert wird; und
im nächsten Schritt Reduzieren der Temperatur der Cycloolefin-Komponente auf eine zweite Temperatur von wenigstens 80 °C und Aufrechterhalten der zweiten Temperatur während wenigstens 20 Minuten, so dass die Brechungsfehler aus der Matrix der Cycloolefin-Komponente entfernt werden;
mit der Maßgabe, dass ein Cycloolefincopolymer mit Polyethylen von der Cycloolefin-Komponente ausgeschlossen ist.

2. Verfahren zur Entfernung von Brechungsfehlern, die in der Matrix einer klaren Cycloolefin-Komponente entstehen, gemäß Anspruch 1, wobei das Verfahren das Halten der Cycloolefin-Komponente auf der zweiten Temperatur in einer trockenen Atmosphäre relativ zu dem ersten Schritt des Erhitzens umfasst.

3. Verfahren zur Entfernung von Brechungsfehlern in der Matrix einer Cycloolefin-Komponente gemäß Anspruch 1, wobei das Verfahren das Erhitzen der Cycloolefin-Komponente auf die erste Temperatur von über 100 °C in Gegenwart von Dampf in einem Autoklaven, dann das Entnehmen der Cycloolefin-Komponente aus dem Autoklaven und das Überführen der Cycloolefin-Komponente in einen Ofen, der eine relativ trockene Atmosphäre aufweist, und das Aufrechterhalten der zweiten Temperatur in dem Ofen während wenigstens 40 Minuten umfasst.

4. Verfahren zur Entfernung von Brechungsfehlern in der Matrix einer Cycloolefin-Komponente gemäß Anspruch 1, wobei das Verfahren das Erhitzen der Cycloolefin-Komponente auf die erste Temperatur in einem Autoklaven, dann das Reduzieren der Temperatur in dem Autoklaven auf die zweite Temperatur und das Aufrechterhalten der Temperatur in dem Autoklaven während wenigstens 30 Minuten umfasst.

5. Verfahren zur Entfernung von Brechungsfehlern in der Matrix einer Cycloolefin-Komponente gemäß Anspruch 1, wobei die Komponente ein trockener medizinischer Behälter ist und das Verfahren Folgendes umfasst: das Erhitzen des trockenen medizinischen Behälters in Gegenwart von Dampf in einem Autoklaven, wobei die erste Temperatur zwischen 120 °C und 130 °C liegt, so dass der trockene medizinische Behälter sterilisiert wird, dann das Reduzieren der Temperatur der trockenen medizinischen Komponente auf die zweite Temperatur, wobei die zweite Temperatur zwischen 80 °C und 120 °C liegt.

6. Verfahren zur Entfernung von Brechungsfehlern in der Matrix einer Cycloolefin-Komponente gemäß Anspruch 1, wobei die Komponente ein medizinischer Behälter ist, der eine Flüssigkeit enthält, und das Verfahren Folgendes umfasst: das Erhitzen des medizinischen Behälters und der Flüssigkeit in Gegenwart von Dampf in einem Autoklaven während wenigstens 30 Minuten, wobei die erste Temperatur zwischen 120 °C und 130 °C liegt, und dann das Reduzieren der Temperatur des Behälters und der Flüssigkeit auf die zweite Temperatur, wobei die zweite Temperatur zwischen 80 °C und 100 °C liegt.

7. Verfahren zur Entfernung von Brechungsfehlern in der Matrix einer Cycloolefin-Komponente gemäß Anspruch 6, wobei das Verfahren das Halten des medizinischen Behälters und der Flüssigkeit auf der zweiten Temperatur in einer relativ trockenen Atmosphäre umfasst, indem man die Einleitung von Wasser in den Autoklaven abbricht.

8. Verfahren zur Entfernung von Brechungsfehlern in der Matrix einer Cycloolefin-Komponente gemäß Anspruch 1, wobei die Komponente ein Spritzenzylinder ist, der aus einem Cycloolefinpolymer oder -copolymer besteht, wobei das Verfahren Folgendes umfasst: das Sterilisieren des Cycloolefinzylinders in einem Autoklaven bei der ersten Temperatur, wobei die erste Temperatur zwischen 120 °C und 130 °C liegt, dann das Reduzieren der Temperatur auf die zweite Temperatur, wobei die zweite Temperatur zwischen 80 °C und 120 °C liegt, und das Aufrechterhalten der zweiten Temperatur.

## Revendications

1. Procédé d'élimination de défauts de réfraction formés dans la matrice d'un composant oléfinique cyclique transparent d'un dispositif médical lors de la stérilisation, ledit procédé comprenant les étapes suivantes effectuées séquentiellement :
premièrement, le chauffage dudit composant oléfinique cyclique à une première température supérieure à 100°C en présence de vapeur pendant au moins 30 minutes afin de stériliser ledit composant oléfinique cyclique ; et
dans l'étape suivante, la réduction de la température dudit composant oléfinique cyclique à une deuxième température au moins égale à 80°C et le maintien de ladite deuxième température pendant au moins 20 minutes afin d'éliminer lesdits défauts de réfraction de la matrice dudit composant oléfinique cyclique ;
à la condition qu'un copolymère d'oléfine cyclique et de polyéthylène soit exclu du composant oléfinique cyclique.

2. Procédé d'élimination de défauts de réfraction formés dans la matrice d'un composant oléfinique cyclique transparent selon la revendication 1, dans lequel ledit procédé comprend le maintien dudit composant oléfinique cyclique à ladite deuxième température dans une atmosphère sèche par rapport à ladite première étape de chauffage.

3. Procédé d'élimination de défauts de réfraction dans la matrice d'un composant oléfinique cyclique selon la revendication 1, dans lequel ledit procédé comprend le chauffage dudit composant oléfinique cyclique à ladite première température supérieure à 100°C en présence de vapeur dans un autoclave, ensuite l'élimination dudit composant oléfinique cyclique dudit autoclave et le transfert dudit composant oléfinique cyclique dans une étuve ayant une atmosphère relativement sèche et le maintien de ladite deuxième température dans ladite étuve pendant au moins 40 minutes.

4. Procédé d'élimination de défauts de réfraction dans la matrice d'un composant oléfinique cyclique selon la revendication 1, dans lequel ledit procédé comprend le chauffage dudit composant oléfinique cyclique à ladite première température dans un autoclave, ensuite la réduction de la température dans ledit autoclave à ladite deuxième température et le maintien de ladite température dans ledit autoclave pendant au moins 30 minutes.

5. Procédé d'élimination de défauts de réfraction dans la matrice d'un composant oléfinique cyclique selon la revendication 1, dans lequel ledit composant est un récipient médical sec et ledit procédé comprend le chauffage dudit récipient médical sec en présence de vapeur dans un autoclave, ladite première température se situant dans la plage de 120°C à 130°C, afin de stériliser ledit récipient médical sec, ensuite la réduction de ladite température dudit composant médical sec à ladite deuxième température, ladite deuxième température se situant dans la plage de 80°C à 120°C.

6. Procédé d'élimination de défauts de réfraction dans la matrice d'un composant oléfinique cyclique selon la revendication 1, dans lequel ledit composant est un récipient médical contenant un liquide et ledit procédé comprend le chauffage dudit récipient médical et du liquide en présence de vapeur dans un autoclave pendant au moins 30 minutes, ladite première température se situant dans la plage de 120°C à 130°C, et ensuite la réduction de la température dudit récipient et du liquide à ladite deuxième température, ladite deuxième température se situant dans la plage de 80°C à 100°C.

7. Procédé d'élimination de défauts de réfraction dans la matrice d'un composant oléfinique cyclique selon la revendication 6, dans lequel ledit procédé comprend le maintien dudit récipient médical et du liquide à ladite deuxième température dans une atmosphère relativement sèche en interrompant l'introduction d'eau dans ledit autoclave.

8. Procédé d'élimination de défauts de réfraction dans la matrice d'un composant oléfinique cyclique selon la revendication 1, dans lequel ledit composant est un fût de seringue formé d'un polymère ou d'un copolymère d'oléfine cyclique, ledit procédé comprenant la stérilisation dudit fût de seringue en oléfine cyclique dans un autoclave à ladite première température, ladite première température se situant dans la plage de 120°C à 130°C, ensuite la réduction de la température à ladite deuxième température, ladite deuxième température se situant dans la plage de 80°C à 120°C, et le maintien de ladite deuxième température.
